# EUROPÄISCHE PATENTSCHRIFT

(11) **EP 2 983 608 B1**
(45) Veröffentlichungstag und Bekanntmachung des Hinweises auf die Patenterteilung: **19.08.2020**
(21) Anmeldenummer: 14711684.2
(22) Anmeldetag: 13.03.2014
(51) Int. Cl.: A61B 34/30, A61B 90/00

(54) **MEDIZINROBOTER**
MEDICAL ROBOT
ROBOT MÉDICAL

(30) Priorität: 08.04.2013 DE 102013005982
(43) Veröffentlichungstag der Anmeldung: 17.02.2016
(73) Patentinhaber: KUKA Deutschland GmbH, 86165 Augsburg (DE)
(72) Erfinder: LOHMEIER, Sebastian, 80639 München (DE)
(74) Vertreter: Schlotter, Alexander Carolus Paul
(86) Internationale Anmeldenummer: PCT/EP2014/000680
(87) Internationale Veröffentlichungsnummer: WO 2014/166573

(56) Entgegenhaltungen:
- WO-A2-2009/004616
- JP-A- 2010 099 403
- US-A- 4 681 413
- US-A1- 2007 173 975
- US-A1- 2011 130 718
- US-A1- 2011 277 775

## Beschreibung

Die vorliegende Erfindung betrifft einen Medizinroboter zum Führen eines minimalinvasiven Instruments sowie eine Medizinroboter-Anordnung mit einem entsprechenden Medizinroboter.

Aus der EP 1 015 068 A1 ist ein Medizinroboter zum Führen eines minimalinvasiven Instruments bekannt. Der Roboter führt das Instrument in einen Patienten durch einen Tubus ein, welcher starr mit einer Kinematik des Roboters verbunden ist.

Die US 2011/277775 A1 betrifft einen sterilen Adapter, ein Abdecktuch einschließlich des Adapters und ein Verfahren zum Abdecken eines Manipulatorarms, wobei der sterile Adapter ein Gehäuse umfasst, das konfiguriert ist, um eine distale Fläche eines Instrumentenmanipulators mit mehreren Manipulatoraktuatorausgängen aufzunehmen, und eine Membranschnittstelle, die an einem distalen Ende des Gehäuses angeordnet ist, wobei die Membranschnittstelle mehrere Aktuatorschnittstellen enthält, wobei der Adapter ferner ein Paar Stützen umfasst, die mit dem Gehäuse gekoppelt sind, wobei das Paar Stützen konfiguriert ist, um ein chirurgisches Instrument mit mehreren Instrumentenaktuatoreingängen zu halten, so dass die mehreren Instrumentenaktuatoreingänge gegenüber entsprechenden Manipulatoraktuatorausgängen mit einer Aktuator-Schnittstelle zwischen jedem entsprechenden Instrumentenaktuatoreingang und Manipulatoraktuatorausgang positioniert sind.

Die US 2007/173975 A1 betrifft einen Roboterarm mit einem parallelen sphärischen Fünflenkerver mit einem entfernten Zentrum der sphärischen Rotation, wobei der Roboterarm eine endoskopische Kamera beweglich trägt, zwei Außenglieder schwenkbar miteinander verbunden sind, mindestens eines der beiden Außenglieder die endoskopische Kamera stützt, zwei Innenglieder schwenkbar mit den beiden Außengliedern verbunden sind, so dass sich die beiden Innenglieder überkreuzen können, die beiden Innenglieder unterstützen beweglich die beiden Außenglieder, ein Grundglied schwenkbar mit den beiden Innengliedern verbunden ist und das Grundglied die beiden Innenglieder beweglich stützt.

Die US 4 681 413 A betrifft eine am Kopf getragene Vorrichtung, an die eine optische Vorrichtung einstellbar gekoppelt ist, wobei die Vorrichtung ein Kopfband, einen Stützbogen für die optische Vorrichtung, die drehbar mit dem Kopfband gekoppelt ist, und einen Verriegelungsmechanismus zum automatischen Verriegeln des Bogens in einer Betrachtungsposition der optischen Vorrichtung und in einer Ruheposition der optischen Vorrichtung umfasst, die ein Schwenken des Bogens zwischen den beiden Positionen zulässt.

Aufgabe der vorliegenden Erfindung ist es, einen verbesserten Medizinroboter bzw. eine verbesserte Medizinroboter-Anordnung zur Verfügung zu stellen.

Diese Aufgabe wird durch die Merkmale der unabhängigen Ansprüche gelöst, die Unteransprüche betreffen vorteilhafte Weiterbildungen.

Ein Medizinroboter nach einer Ausführung der vorliegenden Erfindung weist eine Basis auf. Diese kann inertial bzw. umgebungsfest sein, insbesondere dauerhaft oder lösbar mit einem Boden, einer Wand oder einer Decke eines stationären oder mobilen Operationssaals verbunden sein. Gleichermaßen kann sie dauerhaft oder lösbar mit einem stationären oder mobilen Operationstisch verbunden sein. Die Basis kann auch auf einer mobilen Plattform, insbesondere einer autonomen Plattform, angeordnet sein.

Der Medizinroboter weist einen Instrumenten-Flansch auf, der zum Ankoppeln eines minimalinvasiven Instruments eingerichtet ist. Eine Medizinroboter-Anordnung nach einer Ausführung der vorliegenden Erfindung kann ein oder mehrere, insbesondere unterschiedliche, minimalinvasive Instrumente aufweisen, die - insbesondere abwechselnd - dauerhaft oder lösbar an den Instrumenten-Flansch koppelbar bzw. gekoppelt sind. Der Instrumenten-Flansch kann insbesondere eine Signal- und/oder Energieversorgungs-Schnittstelle zum Signal- und/oder Energieverbinden mit dem Instrument und/oder eine mechanische Schnittstelle zum dauerhaften oder lösbaren Befestigen des Instruments aufweisen.

Ein an den Medizinroboter koppelbares bzw. gekoppeltes minimalinvasives Instrument weist in einer Ausführung einen Instrumentenschaft mit einem Endeffektor auf, der zum Einführen in einen Patienten vorgesehen bzw. eingerichtet ist.

Der Endeffektor kann insbesondere ein Skalpell, eine Schere, Zange oder Klemme, eine optische Aus- und/oder Eintrittsöffnung zum Aussenden und/oder Empfangen von, insbesondere sichtbarer, elektromagnetischer Strahlung, und/oder eine Fluid-Aus- und/oder -Eintrittsöffnung zum Zu- und/oder Abführen von, insbesondere flüssigem und/oder gasförmigem, Fluid aufweisen, insbesondere sein.

Um den Endeffektor bzw. einen Teil des Instrumentenschaftes in den Patienten einzuführen, weist eine Medizinroboter-Anordnung nach einer Ausführung der vorliegenden Erfindung einen Tubus oder mehrere, insbesondere unterschiedliche, Tuben, insbesondere Trokare bzw. Tuben mit einem darin lösbar geführten Stößel mit scharfer oder stumpfer Schneide auf.

Der Medizinroboter kann in einer Ausführung dazu eingerichtet sein, den Instrumentenschaft um einen, insbesondere invarianten bzw. umgebungsfesten, Trokarpunkt zu bewegen, der in einer Ausführung in dem Tubus angeordnet ist. Insbesondere kann der Medizinroboter, insbesondere seine Kinematik, dazu eingerichtet sein, das minimalinvasive Instrument in Richtung einer Instrumentenlängsachse, die mit einer Achsrichtung des Tubus fluchten und/oder den Trokarpunkt enthalten kann, translatorisch zu bewegen, insbesondere, um es (tiefer) in den Patienten einzuführen oder (wenigstens teilweise) aus diesem herauszuziehen. Zusätzlich oder alternativ kann der Medizinroboter, insbesondere seine Kinematik, dazu eingerichtet sein, das minimalinvasive Instrument um die Instrumentenlängsachse zu drehen. Zusätzlich oder alternativ kann der Medizinroboter, insbesondere seine Kinematik, dazu eingerichtet sein, das minimalinvasive Instrument um eine oder zwei Drehachsen zu drehen, die in einer Ausführung, wenigstens im Wesentlichen, senkrecht aufeinander und/oder auf der Instrumentenlängsachse stehen und/oder sich im Trokarpunkt schneiden. Auf diese Weise kann die Kinematik des Medizinroboters einen translatorischen und/oder einen, zwei oder drei Drehfreiheitsgrade des Endeffektors um den Trokarpunkt darstellen.

Zusätzlich oder alternativ weist der Endeffektor in einer Ausführung einen oder mehrere weitere intrakorporale Freiheitsgrade auf, beispielsweise einen, zwei oder mehr Drehfreiheitsgrade um von dem Trokarpunkt beabstandete Drehachsen. Unter einem intrakorporalen Freiheitsgrad wird vorliegend insbesondere ein Freiheitsgrad bzw. eine Bewegungsmöglichkeit des Endeffektors relativ zu dem Instrumentenschaft verstanden.

Ein oder mehrere solche intrakorporale Freiheitsgrade können in einer Ausführung extrakorporal aktuiert sein. Insbesondere kann an dem Instrumentenschaft ein ein- oder mehrachsiger Antrieb zum Aktuieren des Endeffektors angeordnet sein. In einer Weiterbildung ist der Endeffektor mit dem extrakorporalen Antrieb mechanisch, insbesondere durch ein oder mehrere Zug- und/oder Druckmittel und/oder Drehwellen, pneumatisch, hydraulisch und/oder elektrisch gekoppelt.

Der Instrumenten-Flansch ist durch eine aktuierte Kinematik des Medizinroboters verstellbar mit der Basis verbunden. Die Kinematik weist wenigstens sechs, in einer Ausführung wenigstens sieben Gelenke auf. Durch eine Kinematik mit sechs Gelenken kann eine dreidimensionale Lage und eine dreidimensionale Orientierung des Instrumenten-Flanschs dargestellt werden. Insbesondere können die vorstehend erläuterten Freiheitsgrade um den Trokarpunkt durch eine sechsachsige Kinematik dargestellt bzw. aktuiert werden. Eine Kinematik mit sieben oder mehr Gelenken ist redundant und ermöglicht in einer Ausführung vorteilhaft die Darstellung derselben dreidimensionalen Lage und Orientierung des Instrumenten-Flanschs mit unterschiedlichen Posen bzw. unterschiedlichen Gelenkstellungen, insbesondere, um Interferenzen zwischen interagierenden Medizinrobotern zu vermeiden.

In einer Ausführung sind ein oder mehrere, insbesondere alle Gelenke der Kinematik, Drehgelenke, insbesondere mit je einem Drehfreiheitsgrad um eine Gelenk- bzw. Drehachse. In einer Weiterbildung stehen zwei oder mehr, insbesondere alle aufeinanderfolgenden Dreh- bzw. Gelenkachsen der Kinematik senkrecht aufeinander. Hierdurch wird eine vorteilhafte Struktur und Dynamik zur Verfügung gestellt. Die Gelenke sind in einer Ausführung elektrisch, insbesondere elektromotorisch, aktuiert.

Nach einem Aspekt der vorliegenden Erfindung weist der Medizinroboter einen Tubus-Flansch zum, insbesondere lösbaren, Ankoppeln eines Tubus auf, wobei der Tubus-Flansch durch eine zusätzliche bewegliche Gelenkanordnung mit der Kinematik verbunden ist. Der Tubus-Flansch ist in einer Ausführung zum reib- und/oder formschlüssigen dreh- und/oder axialfesten Ankoppeln eines Tubus ausgebildet.

Gegenüber einem freien bzw. ungebundenen Tubus kann ein Tubus, der mit der Kinematik verbunden ist, in einer Ausführung unerwünschte Relativbewegungen des minimalinvasiven Instruments reduzieren, insbesondere den Instrumentenschaft abstützen bzw. lagern. Zusätzlich oder alternativ kann eine Operationspräzision erhöht werden, insbesondere durch eine exaktere Positionierung von Instrument und Tubus zueinander. Zusätzlich oder alternativ kann eine Belastung eines Patienten beim Ein- und/oder Ausführen und/oder Bewegen eines minimalinvasiven Instruments reduziert werden. Zusätzlich oder alternativ kann ein Tubus, der mit der Kinematik verbunden ist, in einer Ausführung das Ein- und Ausführen eines Instruments und/oder einen Instrumentenwechsel vereinfachen, wenn der Tubus nicht durch den Anwender manipuliert werden muss. So kann in einer Ausführung der Instrumentenwechsel vorteilhaft einhändig erfolgen.

Ein starr mit der Kinematik verbundener Tubus wie in der eingangs erläuterten EP 1 015 068 A1 erfordert eine letzte Gelenkachse der Kinematik, die mit der Tubuslängsachse fluchtet und translatorisch ist, um bei einem Ein- bzw. Ausführen des Instruments durch die Kinematik den Tubus patientenfest bzw. invariant zu fixieren. Zudem steht die restliche Kinematik nicht mehr zum Bewegen des Instruments zur Verfügung, da hierdurch zugleich der starr mit der Kinematik verbundene Tubus mitbewegt würde. Entsprechend limitiert ein starr mit der Kinematik verbundener Tubus deren Struktur und Einsatzmöglichkeiten.

Durch eine zusätzliche bewegliche Gelenkanordnung kann der Tubus-Flansch bzw. ein daran gekoppelter Tubus hingegen relativ zu der Kinematik bewegt werden und so in einer Ausführung eine größere Flexibilität bei der Strukturierung und/oder dem Einsatz des Medizinroboters, insbesondere seiner Kinematik, ermöglichen.

So weist in einer Ausführung die Gelenkanordnung einen oder mehrere zusätzliche bzw. kinematikfremde translatorische Freiheitsgrade auf. Insbesondere kann die Gelenkanordnung einen translatorischen Freiheitsgrad in einer Richtung aufweisen, die, wenigstens im Wesentlichen, parallel zu einer Instrumentenlängsachsenrichtung des Instrumenten-Flanschs ist. Unter einer Instrumentenlängsachsenrichtung des Instrumenten-Flanschs wird vorliegend insbesondere die Richtung einer Längsachse eines an den Instrumenten-Flansch gekoppelten minimalinvasiven Instruments verstanden. In einer Ausführung ist der Instrumenten-Flansch zum Ankoppeln eines Instruments in einer einzigen Position eingerichtet, so dass hierdurch auch die Instrumentenlängsachsenrichtung bezüglich des Instrumenten-Flanschs festgelegt ist.

Durch einen solchen Freiheitsgrad kann der Instrumenten-Flansch und mit ihm das angekoppelte minimalinvasive Instrument durch die komplette Kinematik des Medizinroboters in Instrumentenlängsachsenrichtung verstellt werden, was deren Struktur- und/oder Verwendungsmöglichkeiten bzw. -varianten vorteilhaft erhöht.

Insbesondere hierzu kann die Gelenkanordnung einen translatorischen Freiheitsgrad in einer Richtung aufweisen, die, wenigstens im Wesentlichen, senkrecht zu einer instrumentennächsten bzw. letzten Gelenkachse der Kinematik ist, die insbesondere als Drehachse ausgebildet sein kann. In einer anderen Ausführung kann die Richtung eines translatorischen Freiheitsgrads der Gelenkanordnung mit der instrumentennächsten Gelenkachse der Kinematik, insbesondere einer instrumentennächsten Drehachse, einen spitzen Winkel einschließen, der vorzugsweise wenigstens 5° und/oder höchstens 45° Grad beträgt.

Zusätzlich oder alternativ kann die Gelenkanordnung einen translatorischen Freiheitsgrad in einer Richtung aufweisen, die, wenigstens im Wesentlichen, senkrecht zu einer Instrumentenlängsachsenrichtung des Instrumenten-Flanschs und/oder parallel zu einer instrumentennächsten bzw. letzten Gelenkachse der Kinematik ist.

Zusätzlich oder alternativ zu einem oder mehreren translatorischen Freiheitsgraden weist die Gelenkanordnung in einer Ausführung der vorliegenden Erfindung einen oder mehrere zusätzliche bzw. kinematikfremde Drehfreiheitsgrade auf. Insbesondere kann die Gelenkanordnung einen Drehfreiheitsgrad um eine Drehachse aufweisen, die, wenigstens im Wesentlichen, senkrecht zu einer Instrumentenlängsachsenrichtung des Instrumenten-Flanschs ist, insbesondere zwei Drehfreiheitsgrade um Drehachsen, die, wenigstens im Wesentlichen, senkrecht zu der Instrumentenlängsachsenrichtung und/oder zueinander sind.

Durch solche Drehfreiheitsgrade kann in einer Ausführung insbesondere ein Fluchtungsfehler zwischen der Instrumentenlängsachsenrichtung und einem an den Tubus-Flansch angekoppelten Tubus kompensiert werden.

Zusätzlich oder alternativ kann die Gelenkanordnung einen Drehfreiheitsgrad um eine Drehachse aufweisen, die, wenigstens im Wesentlichen, parallel zu einer Instrumentenlängsachsenrichtung des Instrumenten-Flanschs ist, insbesondere mit dieser fluchtet. Insbesondere hierdurch kann in einer Ausführung eine Umorientierung der Kinematik um die Instrumentenlängsachsenrichtung ermöglicht werden, ohne dass der Tubus eine unzulässige Scherbelastung auf den Patienten ausübt.

Zusätzlich oder alternativ kann die Gelenkanordnung, einen Drehfreiheitsgrad um eine Drehachse aufweisen, die, wenigstens im Wesentlichen, senkrecht oder parallel zu einer instrumentennächsten bzw. letzten Gelenk-, insbesondere Drehachse der Kinematik und/oder senkrecht oder parallel zu einer Richtung eines translatorischen Freiheitsgrads der Gelenkanordnung ist.

Zur Darstellung eines translatorischen Freiheitsgrades zusätzlich zu der Kinematik weist die Gelenkanordnung in einer Ausführung wenigstens ein translatorisches kinematikfremdes bzw. zusätzliches Gelenk auf, das nicht Teil der Kinematik bzw. nicht zwischen Instrumenten-Flansch und Basis angeordnet ist. Ein solches translatorisches Gelenk kann insbesondere eine Linearführung aufweisen, insbesondere sein.

Insbesondere zur Darstellung eines Drehfreiheitsgrades zusätzlich zu der Kinematik weist die Gelenkanordnung in einer Ausführung wenigstens ein kinematikfremdes bzw. zusätzliches Drehgelenk auf, das nicht Teil der Kinematik bzw. nicht zwischen Instrumenten-Flansch und Basis angeordnet ist. Ein solches Drehgelenk kann insbesondere einen einzigen Drehfreiheitsgrad oder zwei oder drei Drehfreiheitsgrade aufweisen, insbesondere ein Kardangelenk sein. In einer Ausführung kann auch ein translatorischer Freiheitsgrad durch zwei oder mehr Drehgelenke der Gelenkanordnung, die in einer Ausführung wenigstens im Wesentlichen parallele Drehachsen aufweisen, dargestellt werden bzw. sein.

Ein Gelenk der Gelenkanordnung kann in einer Ausführung gleit- oder wälzgelagert sein, um die Reibung zu reduzieren und/oder die Präzision und/oder maximale Lagerlast zu erhöhen.

Ein oder mehrere Gelenke der Gelenkanordnung sind in einer Ausführung, insbesondere hydraulisch, pneumatisch oder elektrisch, insbesondere elektromotorisch oder elektromagnetisch, aktuierbar bzw. aktuiert, d.h. sie weisen einen Aktuator, beispielsweise einen Elektromotor, zum Bewegen bzw. Verstellen des Gelenks auf. Hierdurch kann in einer Ausführung der Tubus-Flansch aktiv relativ zu der Kinematik verstellt werden.

In einer Ausführung kann ein Endeffektor durch eine Bewegung des Instrumentenschaftes relativ zu einer Kupplung des Instruments zum Ankoppeln an den Instrumenten-Flansch aktuiert werden. Dann kann in einer Ausführung durch die aktuierte Gelenkanordnung bzw. deren Bewegung relativ zur Kinematik bzw. deren Instrumenten-Flansch der Endeffektor eines angekoppelten minimalinvasiven Instruments aktuiert werden.

Zusätzlich oder alternativ sind in einer Ausführung ein oder mehrere Gelenke der Gelenkanordnung passiv, d.h. weisen keinen Aktuator zum Bewegen des Gelenks auf. Hierdurch kann in einer Ausführung eine passive Nachgiebigkeit dargestellt werden. In einer Ausführung sind ein oder mehrere passive Gelenke elastisch gefesselt, insbesondere mechanisch, etwa durch Federn, pneumatisch und/oder hydraulisch. Unter einem elastisch gefesselten Gelenk wird vorliegend insbesondere ein Gelenk verstanden, welches sich nach Wegfall einer äußeren Beaufschlagung selbsttätig in eine entspannte Nulllage zurückzustellen sucht bzw. zurückstellt.

In einer Ausführung sind ein oder mehrere aktive und/oder passive, insbesondere elastisch gefesselte, Gelenke arretierbar, insbesondere, um eine Gelenkstellung zu fixieren. Ein Gelenk kann in einer Ausführung reib- und/oder formschlüssig arretierbar sein, insbesondere durch eine lösbare Klemmverbindung und/oder eine, vorzugsweise vorgespannte, Rastverbindung mit einem oder mehreren beweglichen Elementen, die, vorzugsweise unter Vorspannung, in Aussparungen eingreifen. Ein Element kann in einer Ausführung ein Bolzen oder eine Kugel sein, die durch ein Federmittel in eine Aussparung gedrückt wird, um ein Gelenk zu arretieren. In einer anderen Ausführung kann ein Element ein elastischer Rasthaken sein, der eine Hinterschneidung hintergreift, um ein Gelenk zu arretieren.

In einer Ausführung ist wenigstens ein Gelenk lösbar und/oder mehrfach bzw. wiederholt arretierbar. Es kann in einer Ausführung selbststätig arretieren, wenn eine einzige vorgegebene oder eine von mehreren vorgegebenen Gelenkstellungen erreicht wird, etwa, indem ein vorgespanntes Element bei Erreichen der Gelenkstellung in eine dann fluchtende Aussparung eingreift. Allgemein kann ein aktives oder passives Gelenk in einer Ausführung in genau einer oder mehreren diskreten oder unendlich vielen Gelenkstellungen arretierbar sein.

In einer Ausführung ist wenigstens ein Gelenk der Gelenkanordnung manuell arretierbar und/oder seine Arretierung manuell lösbar. Zusätzlich oder alternativ kann wenigstens ein arretierbares Gelenk der Gelenkanordnung eine aktuierte Arretierung und/oder De-Arretierung aufweisen, insbesondere eine elektromotorische oder - magnetische Fixierung. So kann in einer Ausführung ein Elektromagnet oder -motor ein Element in eine Aussparung ein- und/oder aus dieser ausführen, um ein Gelenk zu arretieren bzw. seine Arretierung zu lösen, d.h. es zu de-arretieren.

In einer Ausführung der vorliegenden Erfindung ist die Gelenkanordnung, insbesondere lösbar, mit einem Endglied der Kinematik bzw. dem Instrumentenflansch verbunden. Hierdurch kann in einer Ausführung die gesamte Kinematik zur Vorpositionierung der Gelenkanordnung genutzt werden.

In einer anderen Ausführung ist die Gelenkanordnung, insbesondere lösbar, mit einem Anfangsglied der Kinematik bzw. der Basis verbunden. Hierdurch steht in einer Ausführung die gesamte Kinematik zur Positionierung des Instrumentenflansches alleine zur Verfügung.

Gleichermaßen kann die Gelenkanordnung, insbesondere lösbar, mit einem Zwischenglied der Kinematik verbunden sein, welches zwei Gelenke der Kinematik miteinander verbindet. Hierdurch steht ein Teil der Kinematik zur gemeinsamen Vorpositionierung von Instrumenten- und Tubus-Flansch zur Verfügung, ein anderer Teil der Kinematik zur Positionierung bzw. Bewegung von Instrumenten- und Tubus-Flansch relativ zueinander.

In einer Ausführung ist der Tubus-Flansch durch die Gelenkanordnung, wenigstens im Wesentlichen, gewichtskompensiert. Hierunter wird insbesondere verstanden, dass die Gewichtskraft des Tubus-Flanschs und der mit ihm verbundenen Gelenkanordnung, wenigstens nicht vollständig, auf den Tubus-Flansch wirkt und sich so über den angekoppelten Tubus nachteilig auf einem Patienten abstützt.

In einer Ausführung kann der Tubus-Flansch durch die Gelenkanordnung passiv gewichtskompensiert sein, insbesondere durch entsprechend elastisch gefesselte passive Gelenke. Zusätzlich oder alternativ kann der Tubus-Flansch durch die Gelenkanordnung aktiv gewichtskompensiert sein, insbesondere durch entsprechend aktuierte aktive Gelenke.

In einer Ausführung weisen ein oder mehrere Gelenke der Gelenkanordnung ein Messmittel, insbesondere einen Sensor, zum Erfassen einer Kraft auf, die auf das bzw. in dem Gelenk wirkt, wobei vorliegend zur kompakteren Darstellung auch ein antiparalleles Kräftepaar, i.e. ein Drehmoment, verallgemeinernd als Kraft bezeichnet wird, das Messmittel also insbesondere auch ein Drehmoment erfassen kann. Durch die Erfassung von Kräften in der Gelenkanordnung kann eine übermäßige Belastung der Gelenkanordnung und/oder der damit verbundenen Kinematik des Medizinroboters und/oder des Tubus und damit des Patienten erfasst werden. Der Medizinroboter, insbesondere seine Gelenkanordnung kann hierauf entsprechend reagieren, beispielsweise nachgeben. Insbesondere kann mittels solcher Messmittel die vorstehend erläuterte aktive Gewichtskompensation implementiert sein.

Nach einem weiteren Aspekt der vorliegenden Erfindung, der mit dem vorstehend erläuterten Aspekt kombiniert sein kann, schneidet eine Instrumentenlängsachsenrichtung des Instrumenten-Flanschs eine instrumentennächste bzw. letzte, als Drehachse ausgebildete, Gelenkachse der Kinematik, unter einem Winkel. Dieser Winkel beträgt in einer Ausführung wenigstens +5° oder -5°, insbesondere wenigstens +15° oder -15°, und/oder höchstens +85° oder -85°, insbesondere höchstens +75° oder -75°. Instrumentenlängsachsenrichtung und Gelenkachse können sich in einer Ausführung in einem Trokarpunkt des Tubus-Flanschs schneiden.

Hierdurch kann in einer Ausführung ein Drehfreiheitsgrad des minimalinvasiven Instruments um den Trokarpunkt im Wesentlichen alleine durch die letzte Gelenkachse der Kinematik dargestellt werden, eine andere Drehachse durch eine planare Bewegung der Kinematik. Zusätzlich oder alternativ kann durch diesen Aspekt eine kompaktere Kinematik, insbesondere Instrumentenanbindung, zur Verfügung gestellt werden, insbesondere verglichen mit dem Aufbau der EP 1 015 068 A1.

In einer Ausführung weist die Kinematik und/oder die Gelenkanordnung eine sogenannte Kettenstruktur auf, bei der jedes Gelenk mit genau einem Vorgängergelenk und höchstens einem Nachfolgergelenk direkt verbunden ist. Kinematik und Gelenkanordnung zusammen können in einer Ausführung entsprechend eine Baumstruktur bilden. Insbesondere kann die Gelenkanordnung an einem Anfangs-, Zwischen- oder Endglied von der Kinematik abzweigen. Hierdurch kann insbesondere die Steuerung des Medizinroboters verbessert werden.

In einer Ausführung weist die Gelenkanordnung genau ein, genau zwei oder genau drei Gelenke auf: durch ein einziges Gelenk kann in einer Ausführung bei sehr kompaktem Aufbau eine wohldefinierte, einfache Beweglichkeit, insbesondere eine Nachgiebigkeit in einer vorgegebenen Richtung, realisiert werden. Zwei oder drei Gelenke können bei weiterhin kompaktem Aufbau eine ausreichende Verstell- bzw. Bewegungsmöglichkeit des Tubus-Flansches relativ zur Kinematik vermitteln.

In einer Ausführung weist die Gelenkanordnung eine sterile Abdeckung auf, die die Gelenkanordnung ganz oder teilweise abdeckt. Dies kann insbesondere bei einer aktuierten Gelenkanordnung zweckmäßig sein, deren Aktuatoren bzw. Gelenkantriebe nicht oder nur bedingt sterilisierbar sind. Die sterile Abdeckung kann in einer Ausführung zusätzlich auch die Kinematik ganz oder teilweise abdecken.

Weitere Vorteile und Merkmale ergeben sich aus den Unteransprüchen und den Ausführungsbeispielen. Hierzu zeigt, teilweise schematisiert:
- Fig. 1:: eine Medizinroboteranordnung mit einem Medizinroboter nach einer Ausführung der vorliegenden Erfindung;
- Fig. 2:: eine Medizinroboteranordnung mit einem Medizinroboter nach einer weiteren Ausführung der vorliegenden Erfindung;
- Fig. 3:: eine Medizinroboteranordnung mit einem Medizinroboter nach einer weiteren Ausführung der vorliegenden Erfindung; und
- Fig. 4:: eine Medizinroboteranordnung mit einem Medizinroboter nach einer Ausführung der vorliegenden Erfindung.

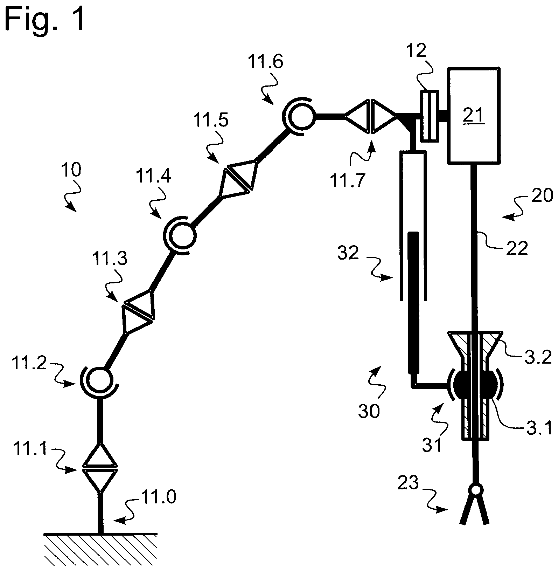

Fig. 1 zeigt eine Medizinroboteranordnung mit einem Medizinroboter 10 nach einer Ausführung der vorliegenden Erfindung.

Der Medizinroboter 10 weist eine inertial bzw. umgebungsfeste Basis 11.0 und einen Instrumenten-Flansch 12 auf, an den ein minimalinvasives Instrument 20 angekoppelt ist. Das minimalinvasive Instrument 20 weist einen Instrumentenschaft 22 mit einem Endeffektor 23 auf, der zum Einführen in einen Patienten (nicht dargestellt) vorgesehen und im Ausführungsbeispiel exemplarisch als Schere ausgebildet ist.

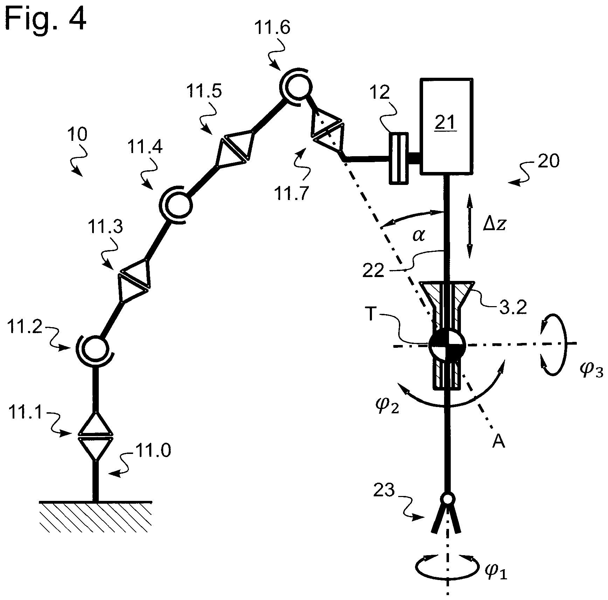

Der Endeffektor 23 weist im Ausführungsbeispiel exemplarisch zwei intrakorporale Freiheitsgrade (Schwenkbewegung der Klingen der Scheren) relativ zu dem Instrumentenschaft 22 auf, d.h. Drehfreiheitsgrade um von einem Trokarpunkt T (vgl. Fig. 4) beabstandete Drehachsen. Zum Aktuieren dieser Freiheitsgrade ist an dem Instrumentenschaft 22 ein Antrieb 21 angeordnet und mit dem Endeffektor 23 mechanisch, beispielsweise durch Zugseile und/oder Zug-/Druckstangen und/oder Drehwellen, gekoppelt.

Das Instrument 20 ist im Ausführungsbeispiel mit seinem Antrieb 21 an den Instrumenten-Flansch 12 gekoppelt, in einer nicht dargestellten Abwandlung kann es auch mit seinem Instrumentenschaft 22, insbesondere einem endeffektorfernen Gehäuse für den Antrieb 21, an den Instrumenten-Flansch 12 gekoppelt sein.

Der Instrumenten-Flansch 12 ist durch eine aktuierte Kinematik des Medizinroboters 10 verstellbar mit der Basis 11.0 verbunden. Die Kinematik weist sieben Drehgelenke 11.1 - 11.7 auf, die jeweils durch einen Elektromotor aktuiert bzw. verstellbar sind. Alle aufeinanderfolgenden Dreh- bzw. Gelenkachsen der Kinematik stehen jeweils senkrecht aufeinander.

Um den Endeffektor und einen Teil des Instrumentenschaftes 22 in den Patienten einzuführen, ist ein Tubus 3.2 vorgesehen.

Der Medizinroboter 10 ist dazu eingerichtet, den Instrumentenschaft 22 um einen invarianten bzw. umgebungsfesten Trokarpunkt T (vgl. Fig. 4) zu bewegen, der in dem Tubus 3.2 angeordnet ist. Insbesondere kann der Medizinroboter 10 durch Verstellen der Gelenke 11.1 - 11.7 seiner Kinematik das minimalinvasive Instrument 20 in Richtung einer Instrumentenlängsachse (vertikal in Fig. 1), die mit einer Achsrichtung des Tubus 3.2 fluchtet und den Trokarpunkt T enthält (vgl. Fig. 4), translatorisch bewegen, insbesondere, um es (tiefer) in den Patienten einzuführen oder (wenigstens teilweise) aus diesem herauszuziehen. Zusätzlich kann der Medizinroboter 10 durch Verstellen der Gelenke 11.1 - 11.7 seiner Kinematik das minimalinvasive Instrument um die Instrumentenlängsachse drehen (vgl. ϕ₁ in Fig. 4) und um zwei weitere Drehachsen drehen (vgl. ϕ₂, ϕ₃ in Fig. 4), die senkrecht aufeinander und auf der Instrumentenlängsachse stehen und sich im Trokarpunkt T schneiden.

Auf diese Weise stellen die Drehgelenke 11.1 - 11.7 einen translatorischen und drei Drehfreiheitsgrade des Endeffektors 23 um den Trokarpunkt T zur Verfügung. Zu diesem treten die zwei vorstehend beschriebenen intrakorporalen Freiheitsgrade, i.e. die Schwenkbewegungen der Scherenklingen, die durch den Antrieb 21 extrakorporal aktuiert sind.

Der Medizinroboter 10 weist einen Tubus-Flansch 3.1 zum lösbaren Ankoppeln des Tubus 3.2 auf. Dieser Tubus-Flansch 3.1 ist durch eine zusätzliche bewegliche Gelenkanordnung 30 mit der Kinematik verbunden.

Die Gelenkanordnung 30 weist im Ausführungsbeispiel der Fig. 1 ein passives translatorisches Gelenk in Form einer Linearführung 32 auf. Zusätzlich weist die Gelenkanordnung ein zusätzliches passives Drehgelenk mit drei orthogonalen Drehfreiheitsgraden in Form eines Kardangelenks 31 auf. Ein Innenring des Kardangelenks 31 bildet den Tubus-Flansch 3.1, an dem der Tubus 3.2, beispielsweise reibschlüssig, angekoppelt ist.

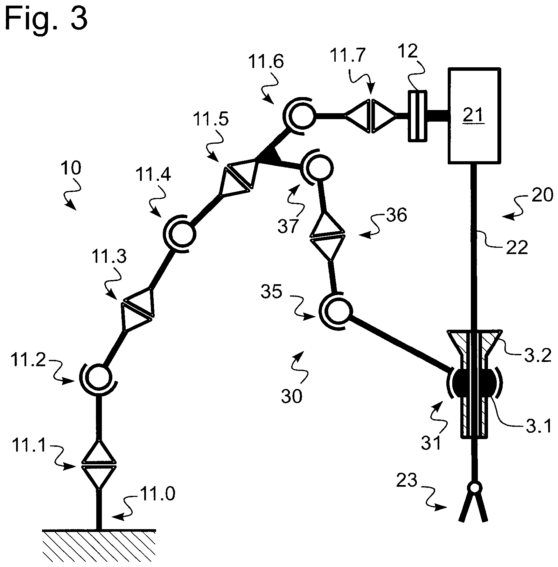

Dadurch weist die Gelenkanordnung 30 einen zusätzlichen, kinematikfremden translatorischen Freiheitsgrad in einer Richtung auf, der parallel zu einer Instrumentenlängsachsenrichtung des Instrumenten-Flanschs 12 (vertikal in Fig. 1) und senkrecht zu einer Drehachse des instrumentennächsten bzw. letzten Gelenks 11.7 der Kinematik ist. In einer nicht dargestellten Abwandlung können ein oder mehrere translatorische Freiheitsgrade auch durch zwei oder mehr Drehgelenke dargestellt sein, wie dies insbesondere nachfolgend mit Bezug auf Drehgelenke 35, 37 der Fig. 3 erläutert ist.

Zusätzlich zu diesem translatorischen Freiheitsgrad weist die Gelenkanordnung durch das Kardangelenk 31 zusätzliche Drehfreiheitsgrade auf, und zwar zwei Drehfreiheitsgrade um Drehachsen, die senkrecht zu der Instrumentenlängsachsenrichtung und zueinander sind, sowie einen Drehfreiheitsgrad um eine Drehachse, die mit der der Instrumentenlängsachsenrichtung des Instrumenten-Flanschs 12 fluchtet. Diese Drehachse ist senkrecht zu der Drehachse des instrumentennächsten Gelenks 11.7 und parallel zu einer Richtung des translatorischen Freiheitsgrads der Gelenkanordnung, während die anderen beiden Drehachsen senkrecht bzw. parallel zur Drehachse des Gelenks 11.7 und senkrecht zu diesem translatorischen Freiheitsgrad sind. In einer Abwandlung kann anstelle eines Kardangelenks 31 ein Drehgelenk mit nur einem Drehfreiheitsgrad um die Längsachse von Instrumentenschaft 22 vorgesehen sein, insbesondere, um ein Umorientieren des Roboters 10 zu ermöglichen, ohne Position und Orientierung des Endeffektors 23 zu verändern. Insbesondere zum Toleranzausgleich kann das Drehgelenk einen oder zwei zusätzliche Drehfreiheitsgrade aufweisen, insbesondere, wie vorstehend beschrieben, als Kardangelenks 31 ausgebildet sein.

Durch diese bewegliche Gelenkanordnung 30 kann die komplette Kinematik 11.1 - 11.7 des Medizinroboters 10 zum Bewegen des Instruments 20 genutzt werden. So kann beispielsweise die redundante Kinematik durch gegensinniges Verdrehen der Gelenke 11.2, 11.4 und 11.6 der Endeffektor in Fig. 1 vertikal verfahren werden, insbesondere bei unbewegtem Gelenk 11.7. Der Tubus-Flansch 3.1 mit angekoppeltem Tubus 3.2 gibt dabei passiv nach. Gleichermaßen kann die redundante Kinematik umorientiert werden, insbesondere um die Instrumentenlängsachsenrichtung. Durch das passive Kardangelenk 31 wird dabei der Tubus 3.2 im Patienten vorteilhaft gering beaufschlagt.

Die passiven Gelenke 31, 32 sind elastisch gefesselt, im Ausführungsbeispiel mechanisch durch Konstantkraftfedern (nicht dargestellt). Dadurch ist der Tubus-Flansch 3.1 passiv gewichtskompensiert.

Wenigstens die Linearführung 32 ist reib- und/oder formschlüssig in einer oder mehreren, insbesondere unendlich vielen Gelenkstellungen arretierbar, beispielsweise durch eine lösbare Klemmverbindung oder eine vorgespannte Rastverbindung (nicht dargestellt). Die Arretierung kann manuell und/oder durch eine elektromotorische oder -magnetische Fixierung erfolgen und/oder gelöst werden.

Die Gelenkanordnung 30 der Fig. 1 ist lösbar mit einem Endglied der Kinematik bzw. dem Instrumentenflansch 12 verbunden. Hierdurch kann in einer Ausführung die gesamte Kinematik zur Vorpositionierung der Gelenkanordnung genutzt werden.

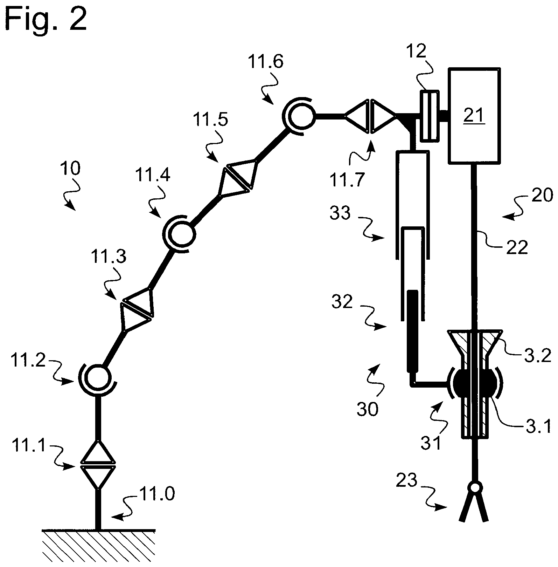

Fig. 2 zeigt eine Medizinroboteranordnung mit einem Medizinroboter nach einer weiteren Ausführung der vorliegenden Erfindung. Mit den anderen Ausführungen übereinstimmende Elemente sind durch gleiche Bezugszeichen bezeichnet, so dass auf deren Beschreibung Bezug genommen und nachfolgend nur auf die Unterschiede eingegangen wird.

Die Gelenkanordnung 30 der Ausführung der Fig. 2 weist zwischen der passiven Linearführung 32 und dem Endglied 12 der Kinematik ein aktives translatorisches Gelenk in Form eines aktuierten Teleskopzylinders 33 auf. Hierdurch kann die Gelenkanordnung (bei eingefahrenem Teleskopzylinder) kompakter gestaut werden. Zusätzlich oder alternativ kann durch den Teleskopzylinder 33 eine aktive Gewichtskompensation der Gelenkanordnung bzw. des Tubus-Flanschs 3.1 und des daran gekoppelten Tubus 3.2 erfolgen. Die passive Linearführung 32 der Fig. 2 bzw. 3 und/oder der aktuierte Teleskopzylinder 33 kann jeweils ein- oder mehrstufig ausgebildet sein, wobei eine Stufe zwei ineinander verschiebbare Teile aufweisen kann. So kann beispielsweise eine zweistufige Linearführung bzw. ein zweistufiger Teleskopzylinder drei konzentrische, wenigstens teilweise ineinander verschiebbare Hülsen aufweisen. Zusätzlich oder alternativ zu ineinander verschiebbaren Teilen kann eine Linearführung bzw. ein Teleskopzylinder auch ein Scherengetriebe oder mehrere serielle Drehgelenke aufweisen, wie sie insbesondere nachfolgend mit Bezug auf Drehgelenke 35, 37 der Fig. 3 erläutert sind. In einer alternativen Ausführung weist die Gelenkanordnung nur einen, insbesondere dreiteiligen, aktuierten Teleskopzylinder auf, in Fig. 2 beispielsweise 32+33. Insofern stellt Fig. 2 gleichermaßen eine Ausführung mit einer passiven Linearführung 32 und einem aktuierten Teleskopzylinder 33 und alternativ auch eine Ausführung mit einem aktuierten, dreiteiligen bzw. zweistufigen Teleskopzylinder 32+33 in einer Figur dar.

Fig. 3 zeigt eine Medizinroboteranordnung mit einem Medizinroboter nach einer weiteren Ausführung der vorliegenden Erfindung. Mit den anderen Ausführungen übereinstimmende Elemente sind durch gleiche Bezugszeichen bezeichnet, so dass auf deren Beschreibung Bezug genommen und nachfolgend nur auf die Unterschiede eingegangen wird.

Die Gelenkanordnung 30 der Ausführung der Fig. 3 ist an einem Zwischenglied der Kinematik zwischen deren Gelenken 11.5 und 11.6 an dieser lösbar befestigt. Sie weist keine translatorischen Gelenke auf. Stattdessen wird insbesondere der vorstehend erläuterte translatorische Freiheitsgrad parallel zur Instrumentenlängsachsenrichtung (vertikal in Fig. 3) durch zwei parallele Drehgelenke 35, 37 der Gelenkanordnung dargestellt. Diese können in einer Weiterbildung kinematisch gekoppelt sein, um den translatorischen Freiheitsgrad mit einem einzigen Antrieb zu aktuieren. Insbesondere, um das letzte Drehgelenk 11.7 der Kinematik zur Bewegung des Instruments 20 nutzen zu können, ist zwischen den parallelen Drehgelenken 35, 37 der Gelenkanordnung ein weiteres Drehgelenk 36 angeordnet, dessen Drehachse auf den Drehachsen der Drehgelenken 35, 37 senkrecht steht.

Die Drehgelenke 35 - 37 der Gelenkanordnung sind durch Elektromotoren (nicht dargestellt) aktuiert. Hierdurch kann zum einen der Tubus-Flansch 3.1 durch die Gelenkanordnung aktiv gewichtskompensiert werden, wie vorstehend mit Bezug auf Fig. 2 erläutert.

Zudem kann der Tubus-Flansch 3.1 aktiv relativ zu der Kinematik 11.1 - 11.7 verstellt werden. Dies kann ausgenutzt werden, um dem Endeffektor 23 zu aktuieren: denkt man sich beispielsweise die beiden Scherenklingen mit dem Tubus 3.2 gekoppelt, bewirkt eine Bewegung des Tubus 3.2 durch die Gelenkanordnung 20 relativ zu dem Instrumenten-Flansch 12 eine Bewegung der Scherenklingen relativ zum Instrumentenschaft 22.

Fig. 4 zeigt eine Medizinroboteranordnung mit einem Medizinroboter nach einer weiteren Ausführung der vorliegenden Erfindung. Mit den anderen Ausführungen übereinstimmende Elemente sind durch gleiche Bezugszeichen bezeichnet, so dass auf deren Beschreibung Bezug genommen und nachfolgend nur auf die Unterschiede eingegangen wird.

Bei dieser Ausführung kann der Tubus 3.2 frei oder an einem Tubus-Flansch (in Fig. 4 nicht dargestellt) angekoppelt sein. Eine Instrumentenlängsachsenrichtung des Instrumenten-Flanschs 12 bzw. des an diesen gekoppelten Instruments 20 schneidet eine Gelenkachse A des instrumentennächsten bzw. letzten Drehgelenks 11.7 im Trokarpunkt T des Tubus 3.2 unter einem Winkel α, der im Ausführungsbeispiel etwa 30° beträgt.

Hierdurch kann der Drehfreiheitsgrad ϕ₃ des minimalinvasiven Instruments 20 um den Trokarpunkt im Wesentlichen durch die letzte Gelenkachse 11.7 der Kinematik dargestellt werden, der andere Drehfreiheitsgrad ϕ₂ durch eine planare Bewegung der Kinematik 11.1 - 11.7. Zusätzlich wird durch diese Abwinkelung eine kompaktere Kinematik, insbesondere Instrumentenanbindung, zur Verfügung gestellt.

In den Ausführungsbeispielen weisen die Kinematik 11.1 - 11. 7 und die Gelenkanordnung 3.1 - 3.4 jeweils eine Kettenstruktur auf, Kinematik und Gelenkanordnung bilden zusammen dementsprechend eine Baumstruktur.

### Bezugszeichenliste

- 10: Medizinroboter
- 11.0: Basis
- 11.1 - 11.7: aktuiertes Drehgelenk (Kinematik)
- 12: Instrumenten-Flansch, Endglied
- 20: minimalinvasives Instrument
- 21: Antrieb
- 22: Instrumentenschaft
- 23: Schere (Endeffektor)
- 3.1: Tubus-Flansch
- 3.2: Tubus
- 30: Gelenkanordnung
- 31: Kardangelenk
- 32: Linearführung (passives translatorisches Gelenk)
- 33: Teleskopzylinder (aktives translatorisches Gelenk)
- 35 - 37: aktives Drehgelenk (Gelenkanordnung)
- T: Trokarpunkt
- A: Gelenkachse

## Patentansprüche

1. Medizinroboter (10) mit:
einer Basis (11.0);
einem Instrumenten-Flansch (12) zum Ankoppeln eines minimalinvasiven Instruments (20), der durch eine aktuierte Kinematik (11.1 - 11.7) verstellbar mit der Basis verbunden ist, wobei die Kinematik wenigstens sechs Gelenke (11.1 - 11.7) aufweist; und
einem Tubus-Flansch (3.1) zum Ankoppeln eines Tubus (3.2),
wobei
der Tubus-Flansch durch eine bewegliche Gelenkanordnung (30), die wenigstens einen kinematikfremden Freiheitsgrad aufweist, mit der Kinematik verbunden ist,
**dadurch gekennzeichnet, dass**
die Gelenkanordnung wenigstens ein elektrisch, pneumatisch oder hydraulisch aktuiertes Gelenk (33 - 37), das einen Aktuator zum Verstellen des Gelenks und ein Messmittel zum Erfassen einer Kraft, insbesondere eines Drehmoments, aufweist, aufweist und der Tubus-Flansch (3.1) durch die Gelenkanordnung aktiv, insbesondere durch entsprechend aktuierte aktive Gelenke, gewichtskompensiert, ist; und/oder dass
die Gelenkanordnung wenigstens ein elastisch gefesseltes passives Gelenk (31, 32), welches sich nach Wegfall einer äußeren Beaufschlagung selbsttätig in eine entspannte Nulllage zurückzustellen sucht, und wenigstens einen kinematikfremden translatorischen Freiheitsgrad in einer Richtung, die parallel zu einer Instrumentenlängsachsenrichtung des Instrumenten-Flanschs ist, aufweist.

2. Medizinroboter nach dem vorhergehenden Anspruch, **dadurch gekennzeichnet, dass** die Gelenkanordnung wenigstens ein arretierbares Gelenk (31 - 37) aufweist.

3. Medizinroboter nach einem der vorhergehenden Ansprüche, **dadurch gekennzeichnet, dass** die Gelenkanordnung, insbesondere lösbar, mit einem End-, Zwischen- oder Anfangsglied der Kinematik verbunden ist.

4. Medizinroboter nach einem der vorhergehenden Ansprüche, **dadurch gekennzeichnet, dass** die Gelenkanordnung wenigstens ein translatorisches und/oder wenigstens ein Drehgelenk (31 - 37) aufweist.

5. Medizinroboter nach einem der vorhergehenden Ansprüche, **dadurch gekennzeichnet, dass** die Gelenkanordnung wenigstens einen translatorischen Freiheitsgrad in einer Richtung aufweist, die, wenigstens im Wesentlichen, parallel oder senkrecht zu einer Instrumentenlängsachsenrichtung des Instrumenten-Flanschs und/oder senkrecht oder parallel zu einer instrumentennächsten Gelenkachse der Kinematik ist.

6. Medizinroboter nach einem der vorhergehenden Ansprüche, **dadurch gekennzeichnet, dass** die Gelenkanordnung wenigstens einen Drehfreiheitsgrad um eine Drehachse aufweist, die, wenigstens im Wesentlichen, senkrecht oder parallel zu einer Instrumentenlängsachsenrichtung des Instrumenten-Flanschs, senkrecht oder parallel zu einer instrumentennächsten Gelenkachse der Kinematik und/oder senkrecht oder parallel zu einer Richtung eines translatorischen Freiheitsgrads der Gelenkanordnung ist.

7. Medizinroboter nach einem der vorhergehenden Ansprüche, **dadurch gekennzeichnet, dass** der Tubus-Flansch (3.1) durch die Gelenkanordnung passiv gewichtskompensiert ist.

8. Medizinroboter (10) nach einem der vorhergehenden Ansprüche, **dadurch gekennzeichnet, dass** eine Instrumentenlängsachsenrichtung des Instrumenten-Flanschs eine instrumentennächsten Drehachse (A) der Kinematik, insbesondere in einem Trokarpunkt (T) eines Tubus-Flanschs (3.1), unter einem Winkel schneidet.

9. Medizinroboter nach einem der vorhergehenden Ansprüche, **dadurch gekennzeichnet, dass** die Kinematik wenigstens sieben Gelenke (11.1 - 11.7) aufweist.

10. Medizinroboter-Anordnung mit einem Medizinroboter (10) nach einem der vorhergehenden Ansprüche und einem minimalinvasiven Instrument (20), welches an den Instrumenten-Flansch angekoppelt ist und einen Instrumentenschaft (22) mit einem Endeffektor (23) zum Einführen in einen Patienten aufweist.

11. Medizinroboter-Anordnung nach dem vorhergehenden Anspruch, **gekennzeichnet durch** eine sterile Abdeckung, die wenigstens die Gelenkanordnung abdeckt.

## Claims

1. A medical robot (10) comprising:
a base (11.0);
an instrument flange (12) for coupling with a minimally invasive instrument (20), which instrument flange (12) is adjustably connected to the base by means of an actuated kinematics system (11.1 - 11.7), wherein the kinematics system comprises at least six joints (11.1 - 11.7); and
a tube flange (3.1) for coupling with a tube (3.2),
wherein
the tube flange is connected to the kinematics system by means of a movable joint arrangement (30) which has at least one degree of freedom which is not due to the kinematics system,
**characterised in that**
the joint arrangement comprises at least one joint (33 - 37) which is actuated electrically, pneumatically or hydraulically, which at least one joint (33 - 37) comprises an actuator for adjusting the joint and a measuring means for detecting a force, in particular a torque, and the tube flange (3.1) is actively weight compensated by means of the joint arrangement, in particular by means of correspondingly actuated active joints; and/or **in that**
the joint arrangement comprises at least one elastically restrained passive joint (31, 32) which automatically seeks to return to a relaxed zero position once it is no longer subjected to an external load, and at least one translatory degree of freedom in a direction which is parallel to a direction of an instrument longitudinal axis of the instrument flange, which translatory degree of freedom is not due to the kinematics system.

2. The medical robot according to the preceding claim, **characterised in that** the joint arrangement comprises at least one lockable joint (31 - 37).

3. The medical robot according to any one of the preceding claims, **characterised in that** the joint arrangement is connected, in particular detachably, to an end link, an intermediate link or an initial link of the kinematics system.

4. The medical robot according to any one of the preceding claims, **characterised in that** the joint arrangement comprises at least one translatory joint and/or at least one rotary joint (31 - 37).

5. The medical robot according to any one of the preceding claims, **characterised in that** the joint arrangement has at least one translatory degree of freedom in a direction which is at least substantially parallel or perpendicular to a direction of an instrument longitudinal axis of the instrument flange and/or perpendicular or parallel to a joint axis of the kinematics system which is nearest to the instrument.

6. The medical robot according to any one of the preceding claims, **characterised in that** the joint arrangement has at least one rotational degree of freedom about an axis of rotation which is, at least substantially, perpendicular or parallel to a direction of an instrument longitudinal axis of the instrument flange, perpendicular or parallel to a joint axis of the kinematics system which is nearest to the instrument and/or perpendicular or parallel to a direction of a translatory degree of freedom of the joint arrangement.

7. The medical robot according to any one of the preceding claims, **characterised in that** the tube flange (3.1) is passively weight compensated by means of the joint arrangement.

8. The medical robot (10) according to any one of the preceding claims, **characterised in that** a direction of an instrument longitudinal axis of the instrument flange intersects, at an angle, an axis of rotation (A) of the kinematics system which is nearest to the instrument, in particular in a trocar point (T) of a tube flange (3.1).

9. The medical robot according to any one of the preceding claims, **characterised in that** the kinematics system comprises at least seven joints (11.1 - 11.7).

10. A medical robot arrangement comprising a medical robot (10) according to any one of the preceding claims and a minimally invasive instrument (20) which is coupled to the instrument flange and which comprises an instrument shaft (22) with an end effector (23) for insertion into a patient.

11. The medical robot arrangement according to the preceding claim, **characterised by** a sterile cover which covers at least the joint arrangement.

## Revendications

1. Robot médical (10) avec :
une base (11.0) ;
une bride d'instrument (12) pour l'accouplement d'un instrument mini-invasif (20), qui est reliée à la base de manière réglable par une cinématique actionnée (11.1 - 11.7), dans lequel la cinématique présente au moins six articulations (11.1 - 11.7) ; et
une bride de tube (3.1) pour l'accouplement d'un tube (3.2),
dans lequel
la bride de tube est reliée à la cinématique par un agencement d'articulation (30) mobile, qui présente au moins un degré de liberté étranger à la cinématique,
**caractérisé en ce que**
l'agencement d'articulation présente au moins une articulation (33 - 37) actionnée de manière électrique, pneumatique ou hydraulique, qui présente un actionneur pour le réglage de l'articulation et un moyen de mesure pour la détection d'une force, en particulier d'un couple, et la bride de tube (3.1) est compensée en poids par l'agencement d'articulation de manière active, en particulier par des articulations actives actionnées en conséquence, et/ou que
l'agencement d'articulation présente au moins une articulation (31, 32) passive enchaînée de manière élastique, laquelle cherche à se remettre après la disparition d'une sollicitation extérieure de manière autonome dans une position zéro détendue, et au moins un degré de liberté de translation étranger à la cinématique dans une direction, qui est parallèle à une direction d'axe longitudinal d'instrument de la bride d'instrument.

2. Robot médical selon la revendication précédente, **caractérisé en ce que** l'agencement d'articulation présente au moins une articulation (31 - 37) pouvant être bloquée.

3. Robot médical selon l'une quelconque des revendications précédentes, **caractérisé en ce que** l'agencement d'articulation est relié, en particulier de manière amovible, à un élément de fin, intermédiaire ou de début de la cinématique.

4. Robot médical selon l'une quelconque des revendications précédentes, **caractérisé en ce que** l'agencement d'articulation présente au moins une articulation de translation et/ou au moins une articulation tournante (31 - 37).

5. Robot médical selon l'une quelconque des revendications précédentes, **caractérisé en ce que** l'agencement d'articulation présente au moins un degré de liberté de translation dans une direction, qui est, au moins sensiblement, parallèle ou perpendiculaire à une direction d'axe longitudinal d'instrument de la bride d'instrument et/ou perpendiculaire ou parallèle à un axe d'articulation proche de l'instrument de la cinématique.

6. Robot médical selon l'une quelconque des revendications précédentes, **caractérisé en ce que** l'agencement d'articulation présente au moins un degré de liberté en rotation autour d'un axe de rotation, qui est, au moins sensiblement, perpendiculaire ou parallèle à une direction d'axe longitudinal d'instrument de la bride d'instrument, perpendiculaire ou parallèle à un axe d'articulation proche de l'instrument de la cinématique et/ou perpendiculaire ou parallèle à une direction d'un degré de liberté de translation de l'agencement d'articulation.

7. Robot médical selon l'une quelconque des revendications précédentes, **caractérisé en ce que** la bride de tube (3.1) est compensée en poids de manière passive par l'agencement d'articulation.

8. Robot médical (10) selon l'une quelconque des revendications précédentes, **caractérisé en ce qu'**une direction d'axe longitudinal d'instrument de la bride d'instrument coupe un axe de rotation proche de l'instrument (A) de la cinématique, en particulier dans un point de trocart (T) d'une bride de tube (3.1), selon un angle.

9. Robot médical selon l'une quelconque des revendications précédentes, **caractérisé en ce que** la cinématique présente au moins sept articulations (11.1 - 11.7).

10. Agencement de robot médical avec un robot médical (10) selon l'une quelconque des revendications précédentes et un instrument mini-invasif (20), lequel est accouplé à la bride d'instrument et présente un arbre d'instrument (22) avec un effecteur terminal (23) à introduire dans un patient.

11. Agencement de robot médical selon la revendication précédente, **caractérisé par** un recouvrement stérile, qui présente au moins l'agencement d'articulation.
